# EUROPEAN PATENT SPECIFICATION

(11) **EP 3 459 569 B1**
(45) Date of publication and mention of the grant of the patent: **14.08.2024**
(21) Application number: 17799589.1
(22) Date of filing: 08.05.2017
(51) Int. Cl.: A61L 27/06, A61L 27/30, A61C 8/00, C25D 11/26, A61L 27/50

(54) **IMPLANT HAVING NANO-PATTERNED GROOVED SURFACE AND METHOD FOR MANUFACTURING SAME**
IMPLANTAT MIT NANO-GEMUSTERTER GENUTETER OBERFLÄCHE UND VERFAHREN ZUR HERSTELLUNG DAVON
IMPLANT PRÉSENTANT UNE SURFACE RAINURÉE À MOTIFS NANOMÉTRIQUES ET SON PROCÉDÉ DE FABRICATION

(30) Priority: 18.05.2016 KR 20160060719
(43) Date of publication of application: 27.03.2019
(73) Proprietor: Korea Electrotechnology Research Institute, Changwon-si, Gyeongsangnam-do 51543 (KR)
(72) Inventor: KIM, Doohun, Changwon-si Gyeongsangnam-do 51475 (KR)
(74) Representative: HGF
(86) International application number: PCT/KR2017/004776
(87) International publication number: WO 2017/200231

(56) References cited:
- WO-A1-2017/047912
- KR-A- 20100 075 032
- KR-A- 20100 075 032
- KR-A- 20110 059 954
- KR-A- 20130 092 855
- KR-A- 20150 011 181
- KR-B1- 101 724 039
- WANG YUE ET AL: "Ordered nano-scale dimple pattern formation on a titanium alloy (Ti-6Al-4V)", AIP ADVANCES, AMERICAN INSTITUTE OF PHYSICS, 2 HUNTINGTON QUADRANGLE, MELVILLE, NY 11747, vol. 2, no. 3, 1 September 2012 (2012-09-01), pages 32101 - 32101, XP012165580, ISSN: 2158-3226, [retrieved on 20120710], DOI: 10.1063/1.4737588

## Description

### Technical Field

The present invention relates to an implant having a nanopatterned dimple surface and a method of preparing the same, and more particularly to an implant having a nanopatterned dimple surface and a method of preparing the same, in which the surface of an implant body is anodized and the anodized surface is then removed to thus form surface dimples, whereby the anodized titanium oxide film may be prevented from being released into a living body due to exfoliation thereof.

### Background Art

An implant has been used to support or attach tissue during treatment or to separate tissue from other tissue, like a molded part such as a membrane, a fixing thin plate, a three-dimensional or spatial part, etc., or a fixing member such as a screw, a pin, a rivet, a tack, etc., which may be implanted into internal organs. A bio-implantable metal for use in manufacturing implants exhibits superior strength, fatigue resistance and moldability compared to other materials such as ceramics, polymers, etc., and is a biomaterial that has been widely used to date in dentistry, orthopedics, and plastic surgery for the purpose of regeneration and treatment of defective parts and damaged parts of the living body. Examples of the bio-implantable metal may include iron (Fe), chromium (Cr), nickel (Ni), stainless steel, a cobalt (Co) alloy, titanium (Ti), a titanium (Ti) alloy, zirconium (Zr), niobium (Nb), tantalum (Ta), gold (Au), silver (Ag), and the like. Among these, stainless steel, titanium, a titanium alloy, or gold, which may exhibit superior corrosion resistance and is stable in human tissue compared to other metal materials, is widely utilized in the human body.

The bio-implantable metal that constitutes the implant that is inserted into the living body has to satisfy the following two conditions. First, biocompatibility should be superior. Specifically, when the metal is used as a replacement for supporting the living body, it should not cause foreign reactions or toxicity to the surrounding tissues. Second, the metal should have a surface that may fuse well with osteogenic cells, which differentiate at various stages, and with the metal surface with which the new bone has been implanted. In order to manufacture such a bio-implantable metal, many attempts have been made to increase the surface area of the metal and to change the surface morphology thereof or perform physical/chemical surface treatment to thus improve bone bondability. Typically, in order to enhance the retention force between bone tissues, formation of thread lines, sandblasting treatment, electrochemical oxidation and the like are performed, and surface treatment, such as anodization, plasma spraying, alkali treatment, ion implantation and the like, is carried out in order to improve the bonding characteristics with the bone along with the formation of a film layer having excellent corrosion resistance.

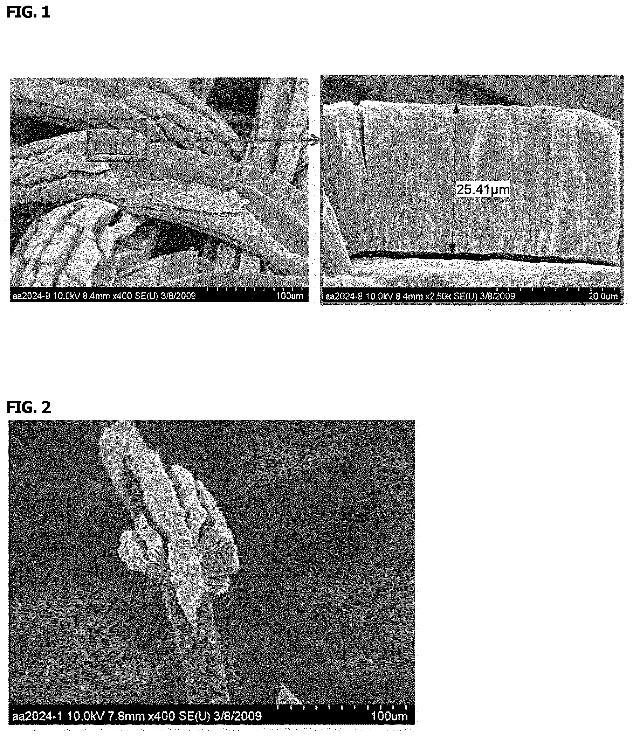

As disclosed in the conventional techniques, namely Korean Patent Application Publication No. 10-2009-0060833, entitled "Implant material through anodization and Method of preparing the same", Korean Patent Application Publication No. 10-2011-0082658, entitled "Surface treatment method of titanium implant and Implant prepared thereby", and Korean Patent Application Publication No. 10-2012-0101748, entitled "Implant surface treatment solution, Surface treatment method using the same and Implant prepared by the method", the metal is anodized to thus form nanotubes on the surface thereof, thereby promoting osseointegration of the metal. However, as in the conventional techniques, the metal oxide on the metal matrix obtained through anodization has poor mechanical strength and may thus be exfoliated from the metal surface during the insertion into the living body to thus be released into the living body. FIG. 1 is SEM images showing the anodized metal surface using a conventional process, in which the separation of the anodized metal oxide from the metal surface can be seen. FIG. 2 is an SEM image showing the exposed internal metal due to easily exfoliated metal oxide. As described above, the anodized metal oxide may be easily exfoliated from the metal surface when undergoing physical force such as bending. In this case, the exfoliated anodized metal oxide may be released into the living body and thus may have a serious influence on the living body. Moreover, the anodization process is problematic in that surface contamination may occur because the electrolyte or chemical components used upon anodization may be left behind. It is difficult to remove residual impurities when the size of the anodized surface pores is decreased to a nano level.

Korean Patent Application Publication No. 10-2008-0075032 describes a method for preparing self-organized anodic titanium oxide nanotube arrays, in which a chemical or mechanical polishing is applied as a pretreatment to planarize the surface of the selected metal.

Wang Yue et. al. in "Ordered nano-scale dimple pattern formation on a titanium alloy (Ti-6Al-4V)" describe formation of an ordered nano-scale dimple pattern formation on a titanium alloy, wherein sizes of a dimples are controlled using parameters of an electropolishing potential, an electrolyte concentration and a surface chemical composition.

### Disclosure

### Technical Problem

Accordingly, the present invention is intended to provide an implant having a nanopatterned dimple surface and a method of preparing the same, in which the surface of an implant body is anodized and the anodized surface is then removed to thus form surface dimples, whereby the anodized titanium oxide film may be prevented from being released into the living body due to exfoliation thereof, and simultaneously, impurities remaining on the surface may be removed upon exfoliation.

In addition, the present invention is intended to provide an implant having a nanopatterned dimple surface and a method of preparing the same, in which a bio-implantable titanium or titanium alloy is used as a material for an implant body, thus exhibiting superior biocompatibility, chemical stability and mechanical stability upon bio-implantation by virtue of dimples formed through surface anodization.

In addition, the present invention is intended to provide an implant having a nanopatterned dimple surface and a method of preparing the same, in which anodization is performed together with a sandblasting or SLA (Sandblasting, Large-grit, Acid etching) process, thus forming roughness having various sizes, including nanopatterns.

### Technical Solution

Therefore, the present invention provides a method of preparing an implant having a nanopatterned dimple surface, comprising: forming titanium oxide nanotubes by anodizing an implant body composed of titanium metal or a titanium alloy; and forming dimples on the surface of the implant body by removing the titanium oxide nanotubes. Therein, the titanium oxide nanotubes are removed in a manner in which the implant body is dipped in a hydrogen peroxide aqueous solution (H₂O₂) and sonicated or in which the implant body is dipped in an organic acid or base aqueous solution.

Here, the forming the titanium oxide nanotubes by anodizing the implant body is preferably performed in a manner in which the implant body is dipped in an electrolyte containing a fluoride (F⁻) ion and thus anodized.

Also, the method of the invention preferably further comprises forming an oxide film having a thickness ranging from 10 nm to 1,000 nm on the surface of the implant body by heat-treating the implant body, after the forming the dimples on the surface of the implant body. Preferably, the dimples have a hemispherical shape and a diameter of 10 nm to 1,000 nm, and the hemispherical dimples further include nanopores having a size of ones of nm on the surface thereof.

The method of the invention preferably further comprises subjecting the surface of the implant body to sandblasting, before the forming the titanium oxide nanotubes, the sandblasting being performed in a manner in which the surface of the implant body is struck by sandblasting media to thus form micro-sized roughness having a size of 50 µm to 500 µm on the surface of the implant body.

The method of the invention preferably further comprises subjecting the implant body to surface treatment through an SLA (Sandblasting, Large-grit, Acid etching) process, before the forming the titanium oxide nanotubes.

In addition, the present invention provides an implant having a nanopatterned dimple surface, configured such that dimples are formed on the surface of an implant body by removing titanium oxide nanotubes formed by anodizing the implant body composed of titanium metal or a titanium alloy. Again, the titanium oxide nanotubes are removed in a manner in which the implant body is dipped in a hydrogen peroxide aqueous solution (H₂O₂) and sonicated or in which the implant body is dipped in an organic acid or base aqueous solution, wherein the dimples include nanopores having a size of ones of nm.

Preferably, the dimples have a hemispherical shape, and a ratio of a diameter and a height of the dimples is 1:0.01 to 0.5. The hemispherical dimples preferably have a diameter of 10 nm to 1,000 nm.

The surface of the implant body is preferably configured to have middle-sized roughness having a size of 1 µm to 50 µm, larger than the dimples, and micro-sized roughness having a size of 50 µm to 500 µm, larger than the middle-sized roughness.

### Advantageous Effects

According to the present invention, the surface of an implant body is anodized and the anodized surface is then removed to thus form surface dimples, whereby the anodized titanium oxide film can be prevented from being released into the living body due to exfoliation thereof and impurities remaining on the surface can be effectively removed.

Furthermore, a bio-implantable titanium or titanium alloy is used as a material for an implant body, and thus the surface area thereof can be maximized upon bio-implantation by virtue of dimples formed through surface anodization, thereby exhibiting superior biocompatibility, chemical stability and mechanical stability, and also, anodization is performed together with a sandblasting or SLA (Sandblasting, Large-grit, Acid etching) process, thereby forming roughness having various sizes, including nanopatterns.

### Description of Drawings

FIGS. 1 and 2 are SEM images of anodized metal oxide according to embodiments of conventional techniques;
FIG. 3 is a flowchart showing a process of preparing an implant having a nanopatterned dimple surface according to a first embodiment of the present invention;
FIG. 4 schematically shows the process of preparing an implant;
FIG. 5 is a flowchart showing a process of preparing an implant according to a second embodiment of the present invention;
FIG. 6 is SEM images showing the implant surface before anodization;
FIG. 7 schematically shows the implant body that is anodized;
FIG. 8 is SEM images showing the implant body having a titanium oxide film formed thereon according to the first embodiment;
FIG. 9 is an SEM image showing the dimples formed on the surface of the implant body after removal of the titanium oxide film according to the first embodiment;
FIGS. 10 and 11 are SEM images showing the dimples and nanopores formed on the surface of the implant body after removal of the titanium oxide film according to the first embodiment;
FIG. 12 is graphs showing the results of XPS surface component analysis of the titanium body before and after nanopatterning according to the first embodiment;
FIG. 13 is an AFM image showing the surface of the implant body after removal of the titanium oxide film according to the first embodiment;
FIG. 14 is SEM images showing the surface of the implant body subjected to sandblasting according to the second embodiment;
FIG. 15 is SEM images showing the dimples formed on the surface of the implant body after removal of the titanium oxide film according to the second embodiment;
FIG. 16 is an AFM image showing the surface of the implant body after removal of the titanium oxide film according to the second embodiment; and
FIG. 17 is SEM images showing the surface of the implant body after SLA surface treatment and then formation and removal of the titanium oxide film according to a third embodiment of the present invention.

### Best Mode

Hereinafter, a detailed description will be given of an implant having a nanopatterned dimple surface and a method of preparing the same according to embodiments of the present invention with reference to the accompanying drawings.

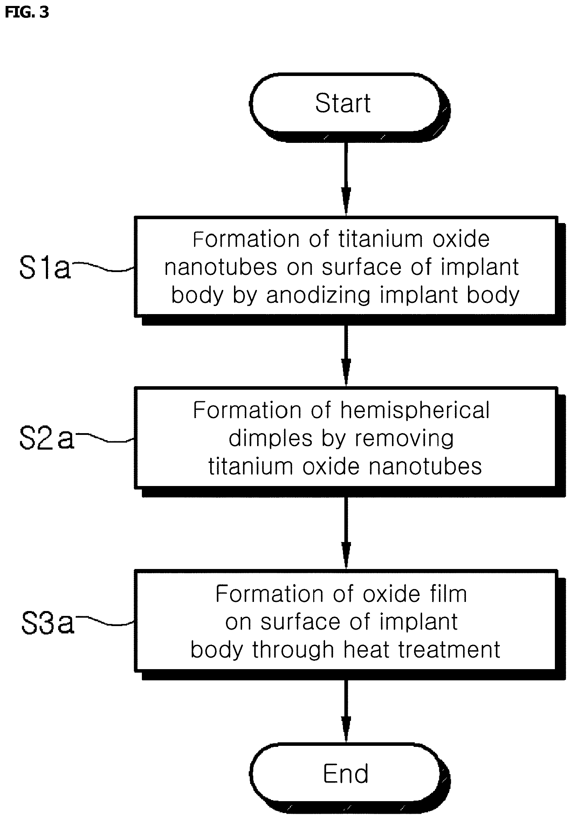

As shown in FIG. 3, the method of preparing an implant according to the first embodiment includes forming titanium oxide nanotubes on the surface of an implant body by anodizing the implant body (S1a).

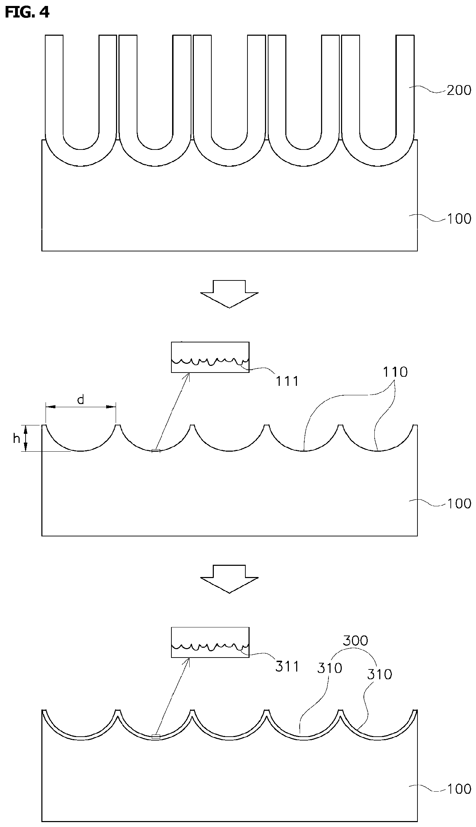

As shown in FIG. 4, an implant body 100 composed of titanium (Ti) or a titanium alloy (Ti alloy) is prepared, and the implant body 100 is anodized, thus forming titanium oxide nanotubes 200 having a titanium nanotube structure shape on the surface thereof. The titanium or titanium alloy is metal suitable for use as a bio-implantable material, and thus the implant body 100 of the present invention is formed exclusively of titanium or a titanium alloy.

Here, the fixture region of the implant body 100 is anodized, and thus the surface of the fixture is formed with dimples. The implant body 100 includes a crown, an abutment and a fixture, the surface of the fixture, which is disposed in the root of the tooth to induce osseointegration, being formed with dimples, whereby the implant body 100 may be used for a long period of time without being detached from the tooth root.

The implant body 100 serving as an anode is dipped in an electrolyte, after which voltage is applied thereto, whereby titanium oxide nanotubes 200 composed of titanium oxide (TiO₂) are formed on the region thereof that is brought into contact with the electrolyte. The titanium oxide nanotubes 200 are provided in the form of a tube on the surface of the implant body 100, and the boundary surface thereof in contact with the implant body 100 is formed in a hemispherical shape like the bottom shape of the tube. The region recessed in the tube form has a diameter ranging from tens of to hundreds of nm, and this diameter may be adjusted by controlling the anodization conditions.

Here, the electrolyte contains a fluoride (F) ion, and the implant body 100 is dipped in the electrolyte and is thus anodized. The electrolyte containing a fluoride ion is preferably prepared by mixing a salt containing a fluoride ion, at least one solvent selected from the group consisting of an inorganic acid, an organic acid, a polymer alcohol and mixtures thereof, and water. Here, the salt containing a fluoride ion is preferably prepared by mixing a salt selected from among hydrogen fluoride (HF), sodium fluoride (NaF), ammonium fluoride (NH₄F) and mixtures thereof with a solvent selected from the group consisting of phosphoric acid (H₃PO₄), sulfuric acid (H₂SO₄), nitric acid (HNO₃), glycerol, ethylene glycol and mixtures thereof.

The halide ion is able to effectively dissolve a metal to thus form corrosion pitting on the metal. However, a halide ion, such as chloride (Cl⁻), bromide (Br⁻), etc., makes it difficult to form a nanotube structure having a straight and uniform size. However, when anodization is carried out in the electrolyte containing a fluoride ion, titanium oxide nanotubes 200 are formed at uniform sizes and intervals. Hence, the electrolyte containing a fluoride ion is used in the present invention.

The titanium oxide nanotubes 200 are removed, thus forming hemispherical dimples 110 on the surface of the implant body 100 (S2a).

The titanium oxide nanotubes 200 formed through anodization are easily exfoliated as shown in FIGS. 1 and 2 when physical force is applied toward the implant body 100 in the living body during or after the insertion of the implant body 100 composed of titanium or a titanium alloy into the living body. As seen in FIGS. 1 and 2, illustrating the metal surface anodized through a typical anodization process according to conventional techniques, even when a slight external force is applied thereto, a metal oxide film is immediately exfoliated. Thus, when the bio-implantable metal obtained through such a conventional technique is inserted into the living body, the metal oxide film is exfoliated and the exfoliated metal oxide film may cause problems such as necrosis of somatic cells and low osseointegration of implants. Furthermore, in the case where nano-sized pores are formed through anodization, it is difficult to effectively remove impurities remaining in the oxide film. With the goal of solving such problems, in the present invention, the titanium oxide nanotubes 200 formed on the implant body 100 are removed.

The titanium oxide nanotubes 200 formed on the surface of the implant body 100 are removed using a physical process or a chemical process, thereby forming hemispherical dimples 110 on the surface of the implant body 100. Even when the titanium oxide nanotubes 200 are removed, the hemispherical dimples 110 formed by the titanium oxide nanotubes 200 are left behind on the surface of the implant body 100. Here, the physical process is preferably performed in a manner in which the titanium oxide nanotubes 200 are dipped in a hydrogen peroxide aqueous solution (H₂O₂) and thus sonicated, and the chemical process is preferably performed in a manner in which the implant body 100 is dipped in an organic acid or base aqueous solution to thus remove the titanium oxide nanotubes 200, but the present invention is not limited thereto. When the titanium oxide nanotubes 200 formed on the surface of the implant body 100 are removed in this way, the resulting implant body 100 is configured such that nano-sized dimples 300 having a hemispherical shape are formed on the surface of the implant body 100 and nanopores 111 having a size of ones of nm, which is smaller than the hemispherical dimples 110, are formed in the hemispherical dimples 110. The hemispherical dimples 110 may be formed at a diameter d of 10 to 1,000 nm, which may be adjusted by controlling electrochemical conditions such as applied voltage, electrolyte, temperature, and the like upon anodization.

As for the ratio of the diameter d and height h of the dimples 110, the ratio of the diameter d and height h of the dimples 110 is preferably 1:0.01 to 0.5, such that the dimples 110 are formed in a hemispherical shape. If the height h of the dimples 110 relative to the diameter d thereof is less than 0.01, surface roughness is low due to excessively low height and osseointegration is not easy. Since the dimples 110 are formed in a hemispherical shape, the height thereof relative to the diameter cannot exceed 0.5. Hence, the ratio of the diameter d and height h in the range of 1:0.01 to 0.5 is most preferable.

The implant body 100 obtained through S1a and S2a may be used immediately because the surface thereof is passivated from metal to metal oxide. When an implant is manufactured through a conventional technique, the process of passivation of titanium into titanium oxide that is suitable for use as a biomaterial and enables oxidation prevention is additionally performed. The passivation process into titanium oxide is conducted by exposing the implant to a high temperature or boiling the implant in hot water. However, in the present invention, anodization is performed, whereby the surface of the implant body 100 may be formed with dimples while passivation into titanium oxide is implemented.

The following step may be further performed as necessary.

An oxide film 300 is formed on the surface of the implant body 100 through heat treatment (S3a).

The implant body 100 having dimples 110 formed on the surface thereof is heat-treated, thus forming an oxide film 300 on the surface of the implant body 100. Here, the oxide film 300 indicates an oxide film 300 including oxidized dimples 310 and oxidized nanopores 311 formed along the dimples 110 and the nanopores 111 of the implant body 100 or an oxide film 300 having nanopores formed therein. The oxide film 300 is preferably a thin film having a thickness of 10 to 1,000 nm. If the thickness of the thin film is less than 10 nm, additional heat treatment is meaningless. On the other hand, if the thickness thereof exceeds 1,000 nm, the oxide film 300 may be stripped from the implant body 100 by an external force.

The oxide film 300 may be amorphous when the surface of the implant body 100 having the dimples 110 is not heat-treated, and may become crystalline upon heat treatment. In this way, the crystalline surface is obtained, thus controlling physicochemical properties such as the hydrophilicity, hardness, strength, and thickness of the oxide film 300. The oxide film 300 includes hemispherical oxidized dimples 310 having oxidized nanopores 311, the heat treatment temperature is preferably set to the range of 200 to 1200°C to form oxidized dimples 310, and the crystalline oxide film 300 is formed at such a temperature. If the heat treatment temperature is lower than 200°C, an amorphous oxide film is formed. On the other hand, if the heat treatment temperature is higher than 1200°C, the implant body 100 may be deformed.

The hemispherical oxidized dimples 310 formed on the oxide film 300 preferably have a diameter of 10 to 1,000 nm. If the diameter of the oxidized dimples 310 is less than 10 nm, bio-binding ability is not high. The oxidized dimples 310 having a diameter exceeding 1,000 nm may be effectively formed even through a chemical or physical process. The diameter of the oxidized dimples 310 may be adjusted by controlling anodization conditions. Also, the surface of the oxidized dimples 310 may be simultaneously formed into a rough surface having fine oxidized nanopores 311 having a size of ones of nm. As described above, heat treatment enables the formation of a crystalline titanium oxide film 300 having a high hardness and a thickness of 10 nm or more, which is thicker than an amorphous titanium oxide film, thereby increasing biocompatibility and hydrophilicity.

Titanium (Ti) is formed into an amorphous titanium oxide (TiO₂) thin film having a thickness of 2 to 5 nm on the surface of the implant body, as the native oxide layer formed after removal of titanium oxide nanotubes 200 resulting from anodization. When heat treatment is conducted at 200 to 1200°C, an anatase crystal phase is formed at about 200°C or more, and a rutile crystal phase oxide film 300 is formed at 700°C or more. The hardness is the order of rutile > anatase > amorphous, and the thickness of the oxide film 300 may be increased to 10 nm or more through heat treatment. As heat treatment is carried out, the hardness of the metal surface having hemispherical oxidized dimples 310 may be increased, and simultaneously, the thickness of the biocompatible oxide film 300 may be increased.

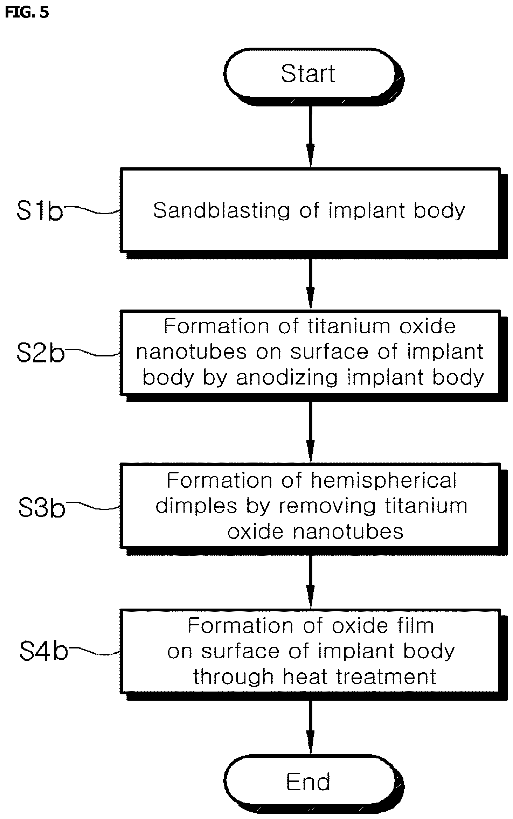

In the first embodiment, the hemispherical dimples 110 are formed on the surface of the implant body 100 through anodization alone, while in the second embodiment, anodization is performed together with sandblasting. As shown in FIG. 5, the method of preparing an implant according to the second embodiment includes subjecting the surface of an implant body to sandblasting (S1b).

Sandblasting is a kind of spraying process, and sandblasting media used therefor may include small glass beads, silicon, beach sand, and metal particles. Sandblasting is performed through striking in a manner in which the sandblasting media are sprayed to the air or are dropped by gravity, thereby forming a fine roughen surface. Specifically, in order to form micro-sized roughness on the surface of the implant body, the surface of the implant body is subjected to sandblasting using sandblasting particles having a size of 1 to 100 µm under a pressure of 0.45 to 0.65 kgf/cm². Here, if the sandblasting pressure is less than 0.45 kgf/cm², the implant may not be efficiently struck by the sandblasting media, and thus it may be impossible to form roughness having a desired size. On the other hand, if the sandblasting pressure exceeds 0.65 kgf/cm², a portion of the implant may be broken. Thereafter, the sandblasting media are completely removed from the surface of the implant body through cleaning. In this way, micro-sized roughness as large as 50 to 500 µm are formed on the surface of the implant body. As for the micro-sized roughness, it is difficult to form roughness having a size of less than 50 µm through sandblasting. On the other hand, if the size exceeds 500 µm, large roughness may be formed, which may have an undesirable influence on the implant body.

Thereafter, the implant body having micro-sized roughness is anodized. Anodizing the implant body (S2b) and forming hemispherical dimples on the surface of the implant body by removing the titanium oxide nanotubes (S3b) remain the same as steps S1a and S2a of the first embodiment, and thus an additional description thereof is omitted. Thereby, hemispherical nano-sized dimples are formed in the micro-sized roughness through anodization.

In some cases, anodization may be performed together with an SLA (Sandblasting, Large-grit, Acid etching) process, in lieu of the sandblasting process in the second embodiment. The SLA process is performed in a manner in which the implant body, the surface of which is formed with roughness having a size of hundreds of µm through sandblasting, is dipped in an acid and etched. When the etching process is performed, middle-sized roughness having a size of 1 to 50 µm, which is smaller than the micro-sized roughness obtained through sandblasting, may be obtained. For the middle-sized roughness, it is difficult to form roughness having a size of less than 1 µm through acid etching. If the size thereof exceeds 50 µm, there is no difference from the large roughness, and thus sandblasting and acid etching, which are additionally performed, may become meaningless.

Base cleaning for neutralizing the acid after acid etching and water or distilled water cleaning for removing the acid and base used for neutralization are sequentially conducted, and cleaning of the particles used for sandblasting is further performed. Thereafter, the anodization process as in the first and second embodiments is performed. In the second embodiment as described above, middle-sized roughness are formed in the micro-sized roughness, and hemispherical nano-sized dimples are formed in the middle-sized roughness.

Below is a detailed description of embodiments of the present invention.

### <Example 1>

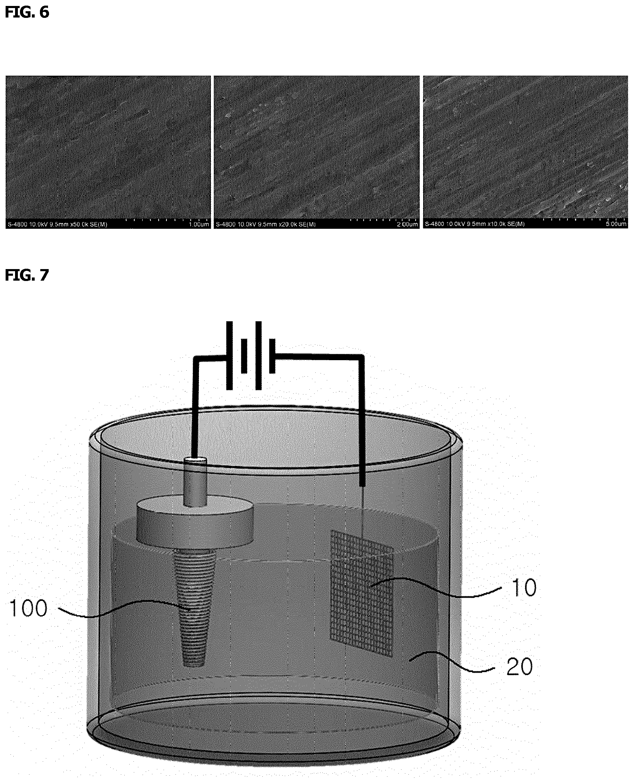

In Example 1, the surface of an implant body composed of titanium metal alone or a titanium alloy is formed with hemispherical dimples through anodization. Here, the surface of the implant body before surface treatment may be confirmed through FIG. 6.

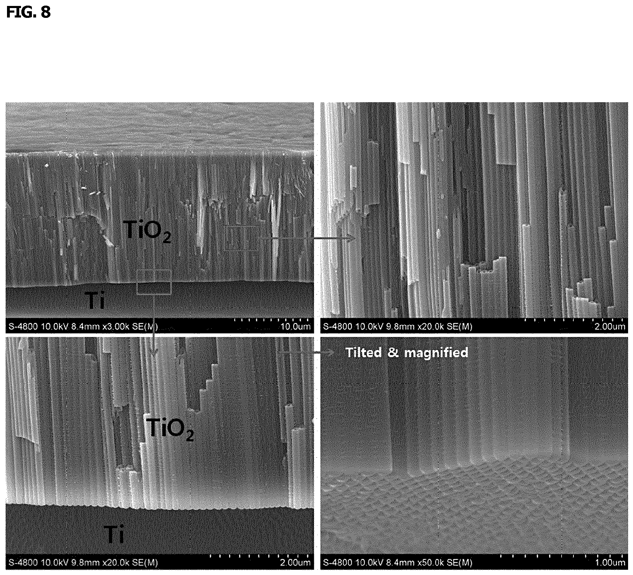

As shown in FIG. 7, direct-current voltage is applied to both of a titanium (Ti) metal implant body 100 serving as an anode and an insoluble platinum (Pt) metal 10 serving as a counter electrode, and anodization is performed, whereby titanium oxide nanotubes may be formed on the metal surface. Before anodization, the implant body 100 is sequentially dipped in ethanol and acetone and cleaned using a sonicator for 2 min each. Thereafter, as shown in FIG. 7, the titanium (Ti) metal implant body 100 to be anodized and the insoluble platinum metal 10 serving as the counter electrode are dipped in an electrolyte 20. Here, the electrolyte 20, obtained by adding a mixture of ethylene glycol and water with 0.01 to 10 wt% of ammonium fluoride (NH₄F), is used, and the temperature of the electrolyte 20 is maintained at 10 to 80°C and a constant voltage of 10 to 200 V is applied for 1 to 300 min, thus obtaining titanium oxide nanotubes having a size of 200 nm or more. The titanium oxide (TiO₂) nanotubes obtained through anodization are shown in FIG. 8, in which the titanium oxide nanotubes can be seen to be formed well.

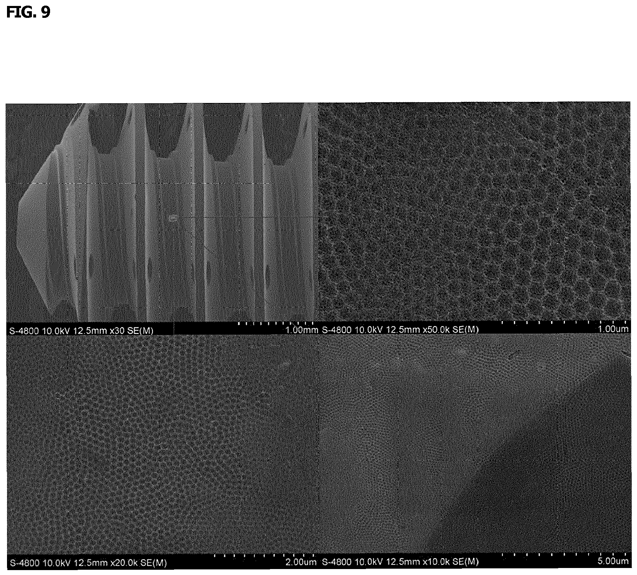

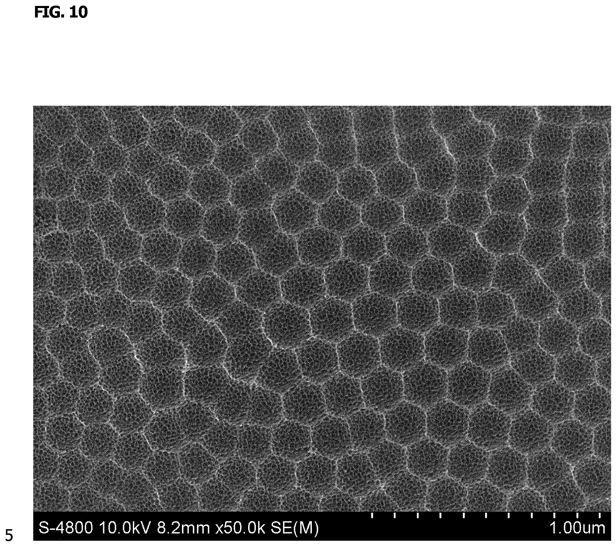

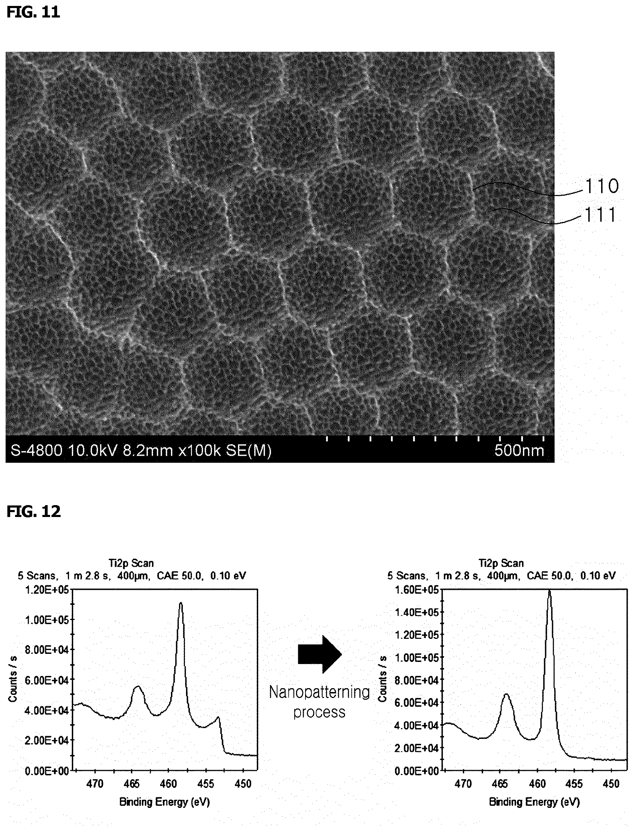

The sample obtained through anodization is dipped in water for 1 hr and cleaned. After completion of the cleaning, in order to remove the titanium oxide nanotubes obtained through anodization from the implant body, the implant body is cleaned in a hydrogen peroxide (H₂O₂) solution using a sonicator at 2 to 100°C for 5 min, cleaned in water using a sonicator for 5 min, and finally cleaned in ethanol using a sonicator for 5 min. The cleaned sample is dried using a hot-air dryer and then stored. Thereby, the titanium oxide nanotubes are removed from the metal surface of the implant body, and the surface thereof is formed with hemispherical dimples, as shown in FIG. 9. When further magnified as shown in FIGS. 10 and 11, fine protrusions can be seen to be formed in the hemispherical dimples. FIG. 12 shows XPS results of the original implant surface and the implant surface after removal of the titanium oxide nanotubes formed through anodization. Based on the XPS results, the implant surface is further oxidized after nanopatterning and is converted into titanium oxide, and also, as is apparent from Table 1 below, impurities such as lead (Pb) are removed. Lead may be left behind on the implant surface during the implant preparation, but may be removed from the implant surface during removal of the titanium oxide nanotubes after anodization, as in the present invention. Here, other impurities, in addition to lead, may be removed therewith.

**[Table 1]**

| Name | Before nanopatterning (At.%) | After nanopatterning (At.%) |
|---|---|---|
| C1s | 47.5 | 47.85 |
| Ca2p | 0.29 | - |
| F1s | 0.33 | 0.41 |
| K2p | - | - |
| Nis | 1.09 | 1.06 |
| O1s | 34.18 | 36.9 |
| Pb4f | 0.13 | - |
| S2p | 0.42 | - |
| Si2p | 1.16 | 0.6 |
| Ti2p | 14.4 | 13.12 |
| Zn2p3 | 0.51 | 0.06 |

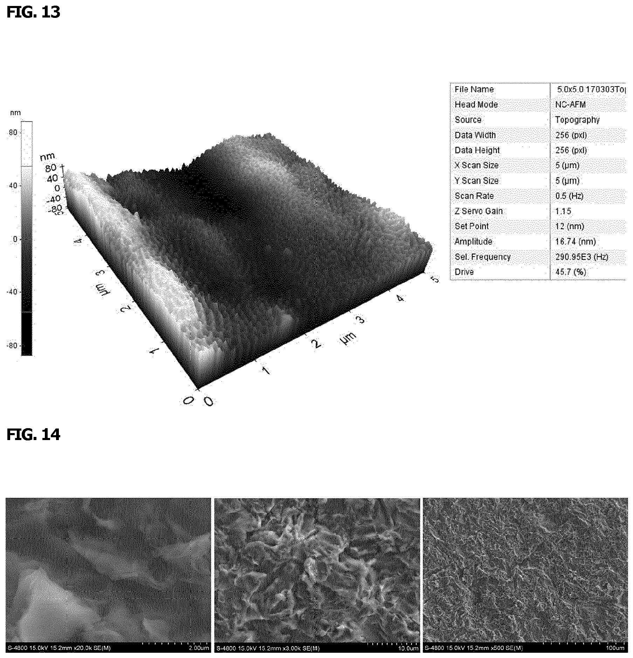

FIG. 13 shows an AFM (Atomic Force Microscope) image of the surface of the implant body after removal of the anodized titanium oxide nanotubes, in which the nano-sized dimples are uniformly formed.

As necessary, the implant body sample is subjected to heat treatment at 200 to 1200°C, whereby the thickness of the titanium oxide film may be increased to the range of about 10 nm to 1,000 nm.

### <Example 2>

In Example 2, an RBM (Resorbable Blast Media) process is performed to form dimples on the surface of an implant body composed of titanium metal alone or a titanium alloy through sandblasting and then anodization.

Specifically, the implant body is fixed so that the position thereof is not moved by pressure, after which the implant body is subjected to surface treatment through sandblasting with the media including calcium phosphate particles having a size of 180 to 425 µm, similar to the size of a grain of sand, using a spray nozzle under appropriate pressure. Here, sandblasting is performed at a pressure of 0.45 to 0.65 kgf/cm². The surface treatment is performed for 10 to 25 sec, and the treated implant body is cleaned for about 5 min using a sonicator. The implant body surface-treated through sandblasting may be confirmed through FIG. 14.

The implant body subjected to sandblasting is anodized. The anodization is performed in the same manner as in Example 1, in which the implant body serving as an anode and platinum as the counter electrode are placed in an electrolyte and then voltage is applied thereto, thus obtaining the implant body having titanium oxide nanotubes having a size of 200 nm or more formed on the surface thereof.

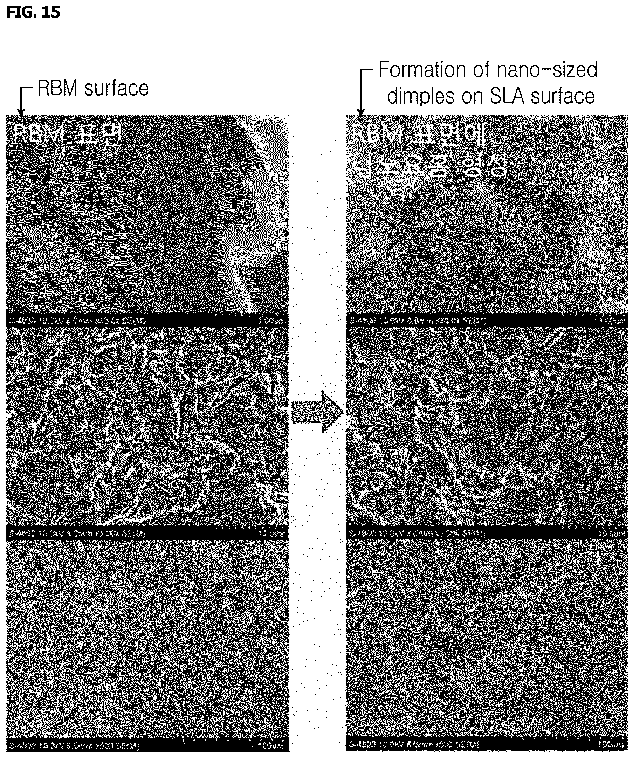

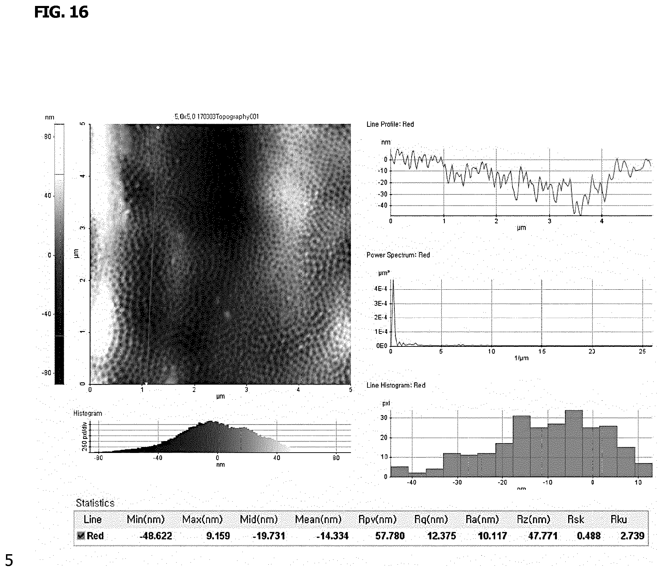

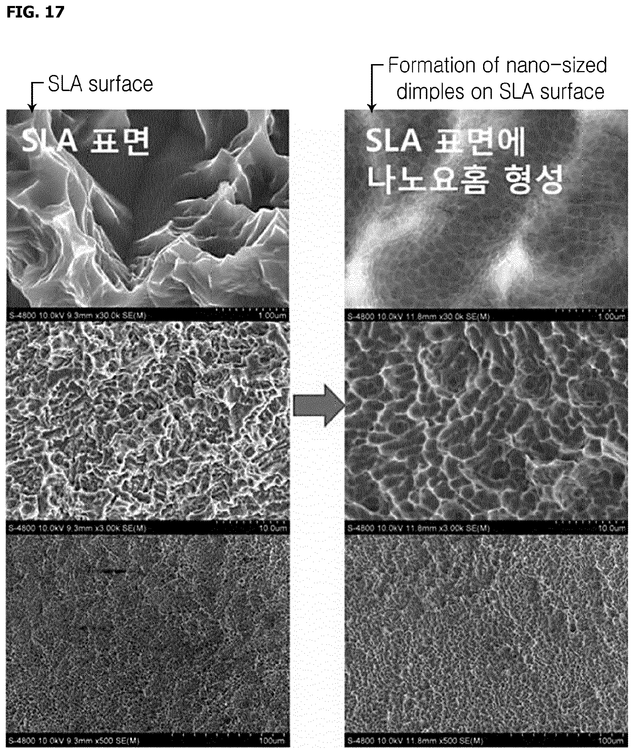

The titanium oxide nanotubes obtained through anodization are sequentially dipped in a hydrogen peroxide aqueous solution, water and ethanol, and are cleaned using a sonicator, thus removing the titanium oxide nanotubes. The implant body having surface roughness thus obtained may be configured such that micro-sized roughness having a size of 100 µm and nano-sized dimples having a size of ones to hundreds of nm are formed on the surface thereof. FIGS. 15 and 16 show the surface of the implant body resulting from sandblasting, anodization and then removal of the titanium oxide nanotubes, in which hemispherical dimples are uniformly formed at a micro-sized roughness. FIG. 17 shows the surface of the implant body having hemispherical dimples, resulting from SLA surface treatment, anodization and then removal of the titanium oxide nanotubes, in which micro-sized roughness and middle-sized roughness are maintained and nanopatterned dimples are formed thereon.

### <Example 3>

In Example 3, an RBM (Resorbable Blast Media) process is performed to form dimples on the surface of an implant body composed of titanium metal alone or a titanium alloy through SLA (Sandblasting, Large-grit, Acid etching) and then anodization.

Specifically, the implant body is fixed so that the position thereof is not moved by pressure, after which the implant body is subjected to surface treatment through sandblasting with aluminum oxide (Al₂O₃) having a size of 100 µm, similar to the size of a grain of sand, using a spray nozzle under appropriate pressure. Here, sandblasting using aluminum oxide (Al₂O₃) is performed at a pressure of 0.45 to 0.65 kgf/cm². The implant body obtained through sandblasting is dipped in an acid and the surface thereof is etched, thus forming roughness having a size of ones of µm. Thereafter, cleaning using a base for neutralizing the acid used for etching and then cleaning using water or distilled water to remove the base used for neutralization are performed. Thereafter, additional cleaning is conducted in order to completely remove the aluminum oxide used for sandblasting.

The implant body subjected to sandblasting and etching is anodized. The anodization is performed in the same manner as in Example 1, in which the implant body serving as an anode and platinum as the counter electrode are placed in an electrolyte and then voltage is applied thereto, thus obtaining the implant body having titanium oxide nanotubes having a size of 200 nm or more formed on the surface thereof.

The titanium oxide nanotubes obtained through anodization are sequentially dipped in water, a hydrogen peroxide aqueous solution and ethanol, and cleaned using a sonicator, thus removing the titanium oxide nanotubes. The implant body having surface dimples thus obtained may be configured such that micro-sized roughness having a size of 100 µm, middle-sized roughness having a size of ones of µm, and nano-sized dimples having a size of ones to hundreds of nm are formed on the surface thereof.

### <Example 4>

In Example 4, an implant body composed of titanium metal alone or a titanium alloy is anodized in the same manner as in Example 1, and dimples are formed on the surface of the implant body, followed by heat treatment, thus increasing the thickness of the thin film.

The anodization is performed in the same manner as in Example 1, in which the implant body serving as an anode and platinum as the counter electrode are placed in an electrolyte and then voltage is applied thereto, thus obtaining an implant body having a nanotube structure having a size of 200 nm or more formed on the surface thereof. The implant body is sequentially dipped in water, a hydrogen peroxide aqueous solution and alcohol and sonicated therein for 5 min each, thus removing the titanium oxide nanotubes. After removal of the titanium oxide nanotubes, drying in an oven at 100°C for 10 min and heat treatment in an electric furnace at 300°C for 1 hr are performed. After the heat treatment, the thickness of the oxide film was increased to about 50 nm.

In such a bio-implantable implant, problems such as somatic necrosis or low osseointegration may be prevented from occurring due to exfoliation of the anodized surface because the surface of the implant body is anodized and then the anodized surface is removed. Furthermore, when a metal biomaterial having hemispherical dimples formed through surface anodization of an implant is implanted, the surface area thereof is maximized, and simultaneously, the surface roughness is increased, thereby exhibiting superior biocompatibility, chemical stability and mechanical stability.

### Industrial Applicability

The present invention pertains to an implant having a nanopatterned dimple surface and a method of preparing the same, and more particularly can be useful in the field of an implant having a nanopatterned dimple surface and a method of preparing the same, in which the surface of an implant body is anodized and the anodized surface is then removed to thus form surface dimples, whereby the anodized titanium oxide film can be prevented from being released into the living body due to exfoliation thereof.

## Claims

1. A method of preparing an implant having a nanopatterned dimple surface, comprising:
forming (S1a) titanium oxide nanotubes by anodizing an implant body composed of titanium metal or a titanium alloy; and
forming (S2a) dimples on a surface of the implant body by removing the titanium oxide nanotubes, wherein the titanium oxide nanotubes are removed in a manner in which the implant body is dipped in a hydrogen peroxide aqueous solution (HzOz) and is sonicated or in which the implant body is dipped in an organic acid or base aqueous solution.

2. The method of claim 1, wherein the forming (S1a) the titanium oxide nanotubes by anodizing the implant body is performed in a manner in which the implant body is dipped in an electrolyte containing a fluoride (F⁻) ion and is anodized.

3. The method of claim 1, further comprising forming (S3a) an oxide film having a thickness ranging from 10 nm to 1,000 nm on the surface of the implant body by heat-treating the implant body, after the forming (S2a) the dimples on the surface of the implant body.

4. The method of claim 1, wherein the dimples have a hemispherical shape and a diameter of 10 nm to 1,000 nm.

5. The method of claim 1, wherein the dimples have a hemispherical shape, and the hemispherical dimples further include nanopores having a size of ones of nm on a surface thereof.

6. The method of claim 1, further comprising subjecting the surface of the implant body to sandblasting (S1b), before the forming (S2b) the titanium oxide nanotubes.

7. The method of claim 6, wherein the sandblasting (S1b) is performed in a manner in which the surface of the implant body is struck by sandblasting media to thus form micro-sized roughness having a size of 50 µm to 500 µm on the surface of the implant body.

8. The method of claim 1, further comprising subjecting the implant body to surface treatment through an SLA (Sandblasting, Large-grit, Acid etching) process, before the forming the titanium oxide nanotubes.

9. An implant having a nanopatterned dimple surface, configured such that dimples are formed on a surface of an implant body by removing titanium oxide nanotubes formed by anodizing the implant body composed of titanium metal or a titanium alloy, wherein the titanium oxide nanotubes are removed in a manner in which the implant body is dipped in a hydrogen peroxide aqueous solution (H₂O₂) and is sonicated or in which the implant body is dipped in an organic acid or base aqueous solution, wherein the dimples further include nanopores having a size of ones of nm.

10. The implant of claim 9, wherein the dimples have a hemispherical shape, and a ratio of a diameter and a height of the dimples is 1:0.01 to 0.5.

11. The implant of claim 9, wherein the dimples have a hemispherical shape, and the hemispherical dimples have a diameter of 10 nm to 1,000 nm.

12. The implant of claim 9, wherein the surface of the implant body is configured to have middle-sized roughness having a size of 1 µm to 50 µm, larger than the dimples, and micro-sized roughness having a size of 50 µm to 500 µm, larger than the middle-sized roughness.

## Patentansprüche

1. Verfahren zur Herstellung eines Implantats mit einer nanostrukturierten Vertiefungsoberfläche, umfassend:
Bilden (S1a) von Titanoxid-Nanoröhrchen durch Anodisieren eines Implantatkörpers, der aus Titanmetall oder einer Titanlegierung besteht; und
Bilden (S2a) von Vertiefungen an einer Oberfläche des Implantatkörpers durch Entfernen der Titanoxid-Nanoröhrchen, wobei die Titanoxid-Nanoröhrchen in einer Weise entfernt werden, bei welcher der Implantatkörper in eine wässrige Wasserstoffperoxidlösung (H₂O₂) getaucht und beschallt wird, oder in einer Weise, bei welcher der Implantatkörper in eine organische Säure oder in eine basische wässrige Lösung getaucht wird.

2. Verfahren nach Anspruch 1, wobei das Bilden (S1a) der Titanoxid-Nanoröhrchen durch Anodisieren des Implantatkörpers in einer Weise durchgeführt wird, bei welcher der Implantatkörper in einen Elektrolyten getaucht wird, der Fluorid-(F⁻)-Ionen enthält, und anodisiert wird.

3. Verfahren nach Anspruch 1, darüber hinaus umfassend, nach dem Bilden (S2a) der Vertiefungen an der Oberfläche des Implantatkörpers, eine Oxidschicht mit einer Dicke von 10 nm bis 1000 nm an der Oberfläche des Implantatkörpers durch Wärmebehandlung des Implantatkörper zu bilden (S3a).

4. Verfahren nach Anspruch 1, wobei die Vertiefungen eine halbkugelige Form und einen Durchmesser von 10 nm bis 1000 nm haben.

5. Verfahren nach Anspruch 1, wobei die Vertiefungen eine halbkugelige Form haben und die halbkugeligen Vertiefungen darüber hinaus Nanoporen mit einer Größe von einigen nm an ihrer Oberfläche aufweisen.

6. Verfahren nach Anspruch 1, darüber hinaus umfassend, vor dem Bilden (S2b) der Titanoxid-Nanoröhrchen die Oberfläche des Implantatkörpers einem Sandstrahlen (S1b) zu unterziehen.

7. Verfahren nach Anspruch 6, wobei das Sandstrahlen (S1b) in einer Weise durchgeführt wird, bei der die Oberfläche des Implantatkörpers durch ein Sandstrahlmittel getroffen wird, um so eine mikrogroße Rauigkeit mit einer Größe von 50 µm bis 500 µm an der Oberfläche des Implantatkörpers zu bilden.

8. Verfahren nach Anspruch 1, darüber hinaus umfassend, vor dem Bilden der Titanoxid-Nanoröhrchen den Implantatkörper einer Oberflächenbehandlung durch einen SLA-Prozess (SLA = Sandstrahlen, grobes Korn, Säureätzen) zu unterziehen.

9. Implantat mit einer nanostrukturierten Vertiefungsoberfläche, die so ausgeführt ist, dass Vertiefungen an einer Oberfläche eines Implantatkörpers durch Entfernen von Titanoxid-Nanoröhrchen gebildet werden, die durch Anodisieren des Implantatkörpers gebildet werden, der aus Titanmetall oder einer Titanlegierung besteht, wobei die Titanoxid-Nanoröhrchen in einer Weise entfernt werden, bei welcher der Implantatkörper in eine wässrige Wasserstoffperoxidlösung (H₂O₂) getaucht und beschallt wird, oder in einer Weise, bei welcher der Implantatkörper in eine organische Säure oder in eine basische wässrige Lösung getaucht wird, wobei die Vertiefungen darüber hinaus Nanoporen mit einer Größe von einigen nm aufweisen.

10. Implantat nach Anspruch 9, wobei die Vertiefungen eine halbkugelige Form haben und das Verhältnis von Durchmesser zu Höhe der Vertiefungen 1:0,01 bis 1:0,5 beträgt.

11. Implantat nach Anspruch 9, wobei die Vertiefungen eine halbkugelige Form und die halbkugeligen Vertiefungen einen Durchmesser von 10 nm bis 1000 nm haben.

12. Implantat nach Anspruch 9, wobei die Oberfläche des Implantatkörpers so ausgeführt ist, dass sie eine mittelgroße Rauigkeit mit einer Größe von 1 µm bis 50 µm, größer als die Vertiefungen, und eine mikrogroße Rauigkeit mit einer Größe von 50 µm bis 500 µm, größer als die mittelgroße Rauigkeit, hat.

## Revendications

1. Procédé de fabrication d'un implant ayant une surface de cavité nano-structurée, comprenant :
former (S1a) des nanotubes d'oxyde de titane par anodisation d'un corps d'implant constitué de titane métallique ou d'un alliage de titane ; et
former (S2a) des cavités sur une surface du corps d'implant en enlevant les nanotubes d'oxyde de titane, sachant que les nanotubes d'oxyde de titane sont enlevés d'une manière dans laquelle le corps d'implant est immergé dans une solution aqueuse de peroxyde d'hydrogène (H₂O₂) et sonorisé, ou d'une manière dans laquelle le corps d'implant est immergé dans un acide organique ou dans une solution aqueuse basique.

2. Le procédé selon la revendication 1, sachant que la formation (S1a) des nanotubes d'oxyde de titane est effectuée par anodisation du corps d'implant d'une manière dans laquelle le corps d'implant est immergé dans un électrolyte contenant des ions fluorure (F⁻) et est anodisé.

3. Le procédé selon la revendication 1, comprenant en outre, après la formation (S2a) des cavités à la surface du corps d'implant, la formation (S3a) d'une couche d'oxyde ayant une épaisseur de 10 nm à 1000 nm à la surface du corps d'implant par traitement thermique du corps d'implant.

4. Le procédé selon la revendication 1, sachant que les cavités ont une forme hémisphérique et un diamètre de 10 nm à 1000 nm.

5. Le procédé selon la revendication 1, sachant que les cavités ont une forme hémisphérique et les cavités hémisphériques comprennent en outre des nanopores de quelques nm à leur surface.

6. Le procédé selon la revendication 1, comprenant en outre, avant la formation (S2b) des nanotubes d'oxyde de titane, la soumission de la surface du corps d'implant à un sablage (S1b).

7. Le procédé selon la revendication 6, sachant que le sablage (S1b) est effectué d'une manière dans laquelle la surface du corps d'implant est frappée par un agent de sablage de manière à former une rugosité micrométrique d'une taille de 50 µm à 500 µm à la surface du corps d'implant.

8. Le procédé selon la revendication 1, comprenant en outre, avant la formation des nanotubes d'oxyde de titane, la soumission du corps d'implant à un traitement de surface par un procédé SLA (SLA = sablage, gros grain, attaque acide).

9. Implant ayant une surface de cavités nano-structurée, qui est conçu de telle sorte que des cavités sont formés sur une surface d'un corps d'implant en enlevant des nanotubes d'oxyde de titane formés par anodisation du corps d'implant constitué de titane métallique ou d'un alliage de titane, sachant que les nanotubes d'oxyde de titane sont enlevés d'une manière dans laquelle le corps d'implant est immergé dans une solution aqueuse de peroxyde d'hydrogène (H₂O₂) et sonorisé, ou d'une manière dans laquelle le corps d'implant est immergé dans un acide organique ou dans une solution aqueuse basique, les cavités comprenant en outre des nanopores de quelques nm.

10. L'implant selon la revendication 9, sachant que les cavités ont une forme hémisphérique et le rapport entre le diamètre et la hauteur des cavités est de 1 :0,01 à 1 :0,5.

11. L'implant selon la revendication 9, sachant que les cavités ont une forme hémisphérique et les cavités hémisphériques ont un diamètre de 10 nm à 1000 nm.

12. L'implant selon la revendication 9, sachant que la surface du corps d'implant est conçue pour avoir une rugosité moyenne d'une taille de 1 µm à 50 µm, supérieure à celle des puits, et une rugosité micrométrique d'une taille de 50 µm à 500 µm, supérieure à celle de la rugosité moyenne.
